(19) Europäisches Patentamt
European Patent Office
Office européen des brevets

(11) EP 4 057 287 A1

(12) **EUROPEAN PATENT APPLICATION**

(43) Date of publication:
**14.09.2022 Bulletin 2022/37**

(21) Application number: **21162297.2**

(22) Date of filing: **12.03.2021**

(51) International Patent Classification (IPC):
***G16B 20/30*** *(2019.01)* ***G16B 40/30*** *(2019.01)*
***G16C 20/20*** *(2019.01)*

(52) Cooperative Patent Classification (CPC):
**G16B 20/30; G16B 40/30; G16C 20/40; G16C 20/90;** G06N 3/00; G16C 20/70

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **UVUE Limited**
**Bury St Edmunds IP33 1NE (GB)**

(72) Inventors:
- **Bagoly, Atilla**
  **Cambridge, CB4 0WS (GB)**
- **Galindo, David**
  **Cambridge, CB4 0WS (GB)**
- **Liu, Jia**
  **Cambridge, CB4 0WS (GB)**
- **Ward, Jonathan**
  **Cambridge, CB4 0WS (GB)**

(74) Representative: **Basck Ltd**
**WeWork**
**50-60 Station Rd**
**Cambridge CB1 2JH (GB)**

(54) **SYSTEM FOR PROCESSING MOLECULAR INFORMATION AND METHOD OF FACILITATING INTER-PARTY COMMUNICATION**

(57) Disclosed is a system for processing molecular information and a method of facilitating inter-party communication relating to molecular fingerprints. The system comprises a server arrangement configured to receive an input of the molecular information, wherein the molecular information comprises information pertaining to molecular structure of at least one molecule; process the molecular information to map the molecular structure of each of the at least one molecule in the input to a molecular fingerprint corresponding thereto using neural networks, wherein the molecular fingerprint is a representation of the at least one molecule in a multi-dimensional space that enables comparison of the at least one molecule with other molecules; encrypt the molecular fingerprints using a symmetric encryption algorithm; and store the encrypted molecular fingerprints in a data repository.

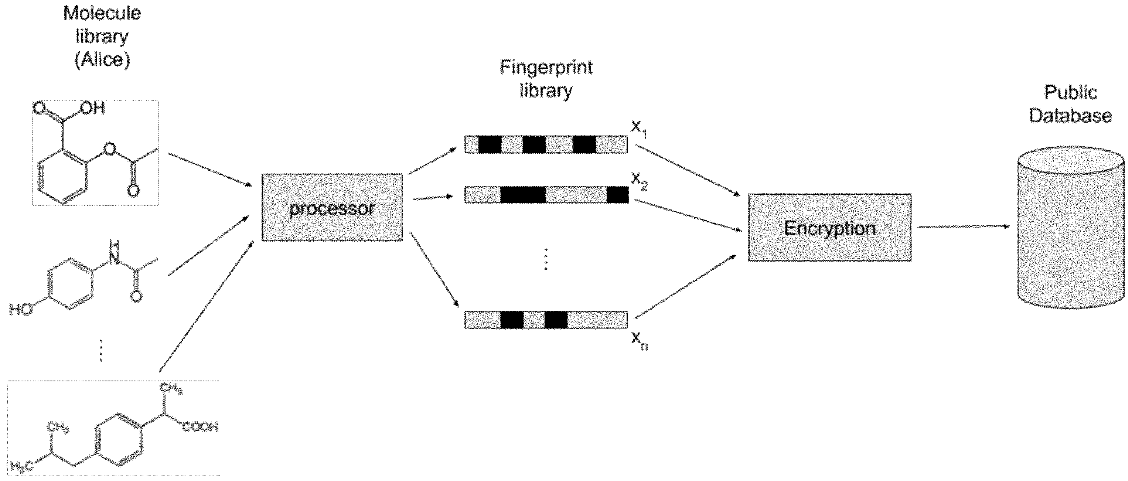


FIG. 2

EP 4 057 287 A1

## Description

TECHNICAL FIELD

The present disclosure relates generally to molecular fingerprinting; and more specifically, to systems for processing molecular information. Furthermore, the present disclosure relates to methods for facilitating inter-party communication relating to molecular fingerprints.

BACKGROUND

Pharmaceutical drug discovery is the process of using small synthesized molecules or compounds to initiate a beneficial change to a biological system to eliminate diseases and increase life expectancy. The efficiency and efficacy of the new pharmaceutical drugs are of utmost importance. Evidently, scientists focus on how a certain disease can be controlled at the molecular level. Computational tools like machine learning (ML), in particular neural networks, play an important role, to accelerate pharmaceutical drug discovery and development. Notably, neural networks help overcome computational complexity, and make compact and informative representations of molecules and/or bioactive compounds such as deoxyribonucleic acid sequence, ribonucleic acid sequence and/or protein sequence by creating data repositories.

However, some pharmaceutical drugs are toxic and cause harmful side effects to a biological body. Notably, databases of such toxic molecules are used to discard those pharmaceutical drugs which were previously found unsuitable, without wasting resources such as time, money, labor and so forth on investigating molecules that have been shown previously to be unstable. However, even with such computational techniques, pharmaceutical companies lose billions of dollars for the development of each new pharmaceutical drug. Much of the expenses are borne at the later stages of evaluation, when it is found that the pharmaceutical drug has harmful side effects. This can be reduced by enabling pharmaceutical companies to share knowledge on toxicity of molecules with each other. Subsequently, the issue of privacy arises, wherein someone can accidentally reveal some sensitive information related to their pharmaceutical company while exchanging molecular information.

In this context, Molecular fingerprints are known tools used for virtual screening and mapping the chemical space. The molecular fingerprint toll is used to map complex three-dimensional structure to a one-dimensional array, but it is possible that distinct molecules and/or bioactive compounds cannot be discriminated from each other as they may have identical molecular fingerprints. Additionally, two molecules and/or bioactive compounds with similar structure might have different fingerprints.

Machine Learning, in particular neural networks offer a way to overcome computational complexity by making compact and informative representations of molecules and/or bioactive compounds. Machine learning has been used to predict a pharmaceutical drug molecule's binding affinity to a specific receptor, or to predict its toxicity. Furthermore, neural networks are used to generate new molecules and/or bioactive compounds with desired properties. Nonetheless, it fails to identify non-identical pharmaceutical drug molecules and/or bioactive compounds which has similar pharmacological effects as of now. A possible solution involves the use of secure multiparty computation (MPC) for sharing of sensitive information among involved parties to prevent revealing unwanted information, but this solution suffers from significant network delays.

Therefore, in light of foregoing discussion, there exists a need to overcome the aforementioned drawbacks.

SUMMARY

The present disclosure seeks to provide a system for processing molecular information. The present disclosure also seeks to provide a method of facilitating inter-party communication relating to molecular fingerprints. An aim of the present disclosure is to provide a solution that overcomes at least partially the problems encountered in prior art, and in particular the problems in the straight-forward application of known cryptographic techniques.

In one aspect, the present disclosure provides a system for processing molecular information, the system comprising a server arrangement configured to

- receive an input of the molecular information, wherein the molecular information comprises information pertaining to molecular structure of at least one molecule;

- process the molecular information to map the molecular structure of each of the at least one molecule in the input to a molecular fingerprint corresponding thereto using neural networks, wherein the molecular fingerprint is a representation of the at least one molecule in a multi-dimensional space that enables comparison of the at least one molecule with other molecules;

- encrypt the molecular fingerprints using a symmetric encryption algorithm with homomorphic properties; and
- store the encrypted molecular fingerprints in a data repository.

In another aspect, an embodiment of the present disclosure provides a method of facilitating inter-party communication relating to molecular fingerprints, the method comprising

- receiving a search query from a first party, the search query comprising molecular information pertaining to molecular structure of at least one molecule;
- processing the molecular information to map the molecular structure of a given molecule in the search query to a molecular fingerprint corresponding thereto using neural networks;
- matching the molecular fingerprint corresponding to the search query from the first party to an existing encrypted molecular fingerprint from a second party stored in a data repository;
- performing operations on the existing encrypted molecular fingerprint for generating a ciphertext, wherein the ciphertext enables multi-party computation between the first party and the second party by computing a function on the matched molecular fingerprints.

In yet another aspect, the present disclosure provides a method of (for) processing molecular information, the method comprising

- receiving an input of the molecular information, wherein the molecular information comprises information pertaining to molecular structure of at least one molecule;
- processing the molecular information to map the molecular structure of each of the at least one molecule in the input to a molecular fingerprint corresponding thereto using neural networks, wherein the molecular fingerprint is a representation of the at least one molecule in a multi-dimensional space that enables comparison of the at least one molecule with other molecules;
- encrypting the molecular fingerprints using a symmetric encryption algorithm with homomorphic properties; and
- storing the encrypted molecular fingerprints in a data repository.

In yet another aspect, the present disclosure provides a software product recorded on machine-readable non-transient data storage media, wherein the software product is executable upon computing hardware to implement any of the methods as mentioned above.

Embodiments of the present disclosure substantially eliminate or at least partially address the aforementioned problems in the prior art, and enable a technique for molecular fingerprinting that can overcome computational complexity of matching ciphertexts computed with homomorphic encryption.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:

FIG. 1 is an illustration of representation of complex three-dimensional structures of molecules using simplified representations, in accordance with an embodiment of the present disclosure;

FIG. 2 is a schematic illustration of encryption algorithm for database, in accordance with an embodiment of the present disclosure;

FIG. 3 is a schematic illustration of multi-party computation of molecular fingerprint matching, in accordance with an embodiment of the present disclosure; and

FIG. 4 is an illustration of representation of the calculation rules of a recurrent neural network (RNN), in accordance with an embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

In one aspect, the present disclosure provides a system for processing molecular information, the system comprising a server arrangement configured to

- receive an input of the molecular information, wherein the molecular information comprises information pertaining to molecular structure of at least one molecule;
- process the molecular information to map the molecular structure of each of the at least one molecule in the input to a molecular fingerprint corresponding thereto using neural networks, wherein the molecular fingerprint is a representation of the at least one molecule in a multi-dimensional space that enables comparison of the at least one molecule with other molecules;
- encrypt the molecular fingerprints using a symmetric encryption algorithm with homomorphic properties; and
- store the encrypted molecular fingerprints in a data repository.

In another aspect, the present disclosure provides a method of facilitating inter-party communication relating to molecular fingerprints, the method comprising

- receiving a search query from a first party the search query comprising molecular information pertaining to molecular structure of at least one molecule;
- processing the molecular information to map the molecular structure of a given molecule in the search query to a molecular fingerprint corresponding thereto using neural networks;
- matching the molecular fingerprint corresponding to the search query from the first party to an existing encrypted molecular fingerprint from a second party stored in a data repository;
- performing operations on the existing encrypted molecular fingerprint for generating a ciphertext, wherein the ciphertext enables multi-party computation between the first party and the second party by computing a function on the matched molecular fingerprints.

The system and method of the present disclosure aim to provide a molecular fingerprinting technique that overcomes the prior difficulties and limitations of molecular fingerprinting, and in particular for their usage in privacy-preserving collaborative drug discovery. Notably, the present disclosure enables distinct molecules to be discriminated from each other even if they possess identical molecular fingerprints. The system described herein enables proper representation of molecular fingerprint space for two molecules with similar structures leading to distinctive molecular fingerprints. Specifically, the distinctive molecular fingerprint for two molecules with similar molecular fingerprints are obtained by proper representation of molecular fingerprint space. Herein, the molecular fingerprint space is the structural distance between the molecules. Moreover, such molecular representation further resolves issues with sparse molecular fingerprints high dimensionality. Notably, neural network models usually build a representation of the ligands in a lower-dimensional nonlinear space with additional constraints. Hence, small movements in this space yield molecules that constitute potential drug targets. Furthermore, neural networks enable inexact matching of molecules to identify close but non-identical molecules, which have similar pharmacological effects.

Notably, cryptographic techniques based on homomorphic encryption, i.e. encryption techniques with homomorphic properties, and multi-party communication are used to prevent sharing of sensitive information while exchanging molecular information between two parties. Homomorphic encryption is a known technique which includes multiple types of encryption schemes that enable the performance of different classes of computations over encrypted data. An Example of a system with homomorphic properties may be additive homomorphic encryption using Paillier encryption scheme. Furthermore, the cryptographic techniques also minimize the network delays experienced during multi-party communication.

Pursuant to the embodiments of the present disclosure, the system described herein may be employed for improving efficiency of the processes relating to drug discovery, for example by pharmaceutical industries. Usually, small synthesized molecules are used for the process of drug discovery that initiate a beneficial change to a biological system so that a disease is eradicated or a symptom is alleviated. The development of each new drug is estimated to cost more than a billion dollars, with much expense being caused by molecules being found to have harmful side-effects at later stages of the evaluation process. Therefore, the pharmaceutical industries maintain databases of toxic molecules which cause excessive harm or toxicity to the biological system. In order to avoid wasting resources on investigating molecules previously shown to be harmful or toxic, the present disclosure enables pharmaceutical industries to share information present in their databases on a toxicity of a molecule with other similar pharmaceutical industries in a secure manner.

Throughout the present disclosure, the term *"server arrangement"* refers to structure and/or module that includes programmable and/or non-programmable components configured to store, process and/or share information. Specifically, the server arrangement includes any arrangement of physical or virtual computational entities capable of enhancing information to perform various computational tasks. Furthermore, it should be appreciated that the server arrangement may be a single hardware server and/or plurality of hardware servers operating in a parallel or distributed architecture. In an example, the server arrangement may include components such as memory, at least one processor, a network adapter and the like, to store, process and/or share information with other entities, such as a pharmaceutical company or a data repository for receiving and sending search queries of molecular information.

The server arrangement is configured to receive an input of the molecular information, wherein the molecular information comprises information pertaining to molecular structure of at least one molecule. In an example, the molecule may be a biopolymer sequence such as deoxyribonucleic acid sequence, ribonucleic acid sequence and/or protein sequence. Notably, the information pertaining to the molecular structure comprises information relating to location of atoms, functional groups or ions relative to one another therein and number and location of chemical bonds. Additionally, the molecular information can comprise partial or complete molecular information.

Optionally, the server arrangement is configured to receive the input from a user or a database storing the molecular information. In an instance, the input may be received from a user, wherein the user may employ a computing device communicably coupled with the server arrangement, to provide the molecular information. The computing device may be a cellular phone, personal digital assistant (PDAs), handheld device, laptop computer, personal computer. Alternatively, the server arrangement is communicably coupled to the database storing the molecular information.

The server arrangement is configured to process the molecular information to map the molecular structure of each of the at least one molecule in the input to a molecular fingerprint corresponding thereto using neural networks, wherein the molecular fingerprint is a representation of the at least one molecule in a multi-dimensional space that enables comparison of the at least one molecule with other molecules. Notably, in molecular fingerprinting, complex three-dimensional structure of a molecule is mapped, according to its functional groups or a more complex deterministic algorithm, to a simple one-dimensional vector molecular fingerprint that can be matched against the database. Significantly, the simplest and most efficient approach to establish exact molecular identity is to serialise the representation of the molecule by using any arbitrary but deterministic encoding scheme and then compute a cryptographic hash of the serialised data. Herein the deterministic encoding scheme is a suite of cryptographic algorithm which always produces the same ciphertext for a given plaintext and key, even over separate executions of the encryption algorithm. Additionally, herein cryptographic hash is an algorithm that takes an arbitrary amount of input and produces a fixed-size output of ciphertext. This approach works efficiently and well for exact matching but is not suitable for inexact matching of molecules. Additionally, neural network-based molecular embedding extends the simple molecular fingerprint to generate an embedding or encoding of the molecule that can be queried as a molecular fingerprint similar to the simpler molecular fingerprinting approach.

In an embodiment, autoencoders are unsupervised neural networks that learn how to efficiently compress and encode data. Subsequently, it learns how to reconstruct the data back from the reduced encoded representation to a representation that is as close to the original input as possible. Furthermore, the neural networks for mapping the molecular structure comprise variational autoencoders wherein the molecular information is represented as distributions over the encoded space instead of single points.

Optionally, the present disclosure comprises adversarial autoencoders wherein autoencoders encodes the input into a low dimensional embedding space and a discriminator network predicts whether the embedding is from an encoder or from a normal distribution. Herein, the dimensional embedding space is the data embedded after dimensionality reduction. Additionally, the discriminator network is a classifier which classifies the generated data according to the molecular fingerprints.

Optionally, variational autoencoders are a special type of autoencoders which overcome the overfitting problem associated with autoencoders. The molecular inputs are represented as distributions over the encoded space instead of single points. The encoder network learns two low dimensional vector molecular fingerprints embedding of the molecular input, these are considered as mean and standard deviation of a normal distribution. With these parameters sampling can be performed from the distribution and be used as molecular input for the decoder network. The loss comprises two parts, reconstruction loss such as mean-squared error and the Kullback-Leibler divergence of the distribution and normal distribution with no mean, and one standard deviation

$$2KL(N(\mu, \Sigma)|N(0,1)) = \quad Tr(\Sigma) + \mu^T\mu - logdet\Sigma - k.$$

Yet more optionally, adversarial autoencoders makes the distributions in latent space continuous. Hereupon, an autoencoder network is used which encodes the molecular input into a low dimensional embedding space and a discriminator network is used which predicts if an embedding is coming from the encoder or from a normal distribution. Hence, the reconstruction and discriminator loss help the autoencoder to build a continuous, distributed latent space by penalizing the encoder network for creating outputs different from the molecular input. Herein, the discriminator loss is the penalization that a discriminator network applies to itself for misclassifying a real input as fake, or a fake input as real.

The server arrangement is configured to encrypt the molecular fingerprints using a symmetric encryption algorithm. In particular, the server arrangement is configured to encrypt the molecular fingerprints using a symmetric encryption algorithm with homomorphic properties. Thereafter, once the molecule representations have been generated using molecular fingerprinting, either with or without using a neural network for greater expressive power, secure multiparty computation (MPC) protocols can be used. Notably, MPC is used by multiple parties to jointly compute a function over their inputs while keeping those inputs private.

In an embodiment, for performing MPC, privacy-preserving protocols are used for computing molecular fingerprint similarity wherein MPC, in particular two-party computation protocol based on garbled circuits and/or secret sharing, can perform operations such as addition and/or subtraction and/or multiplication and/or division and/or comparison on encrypted private molecular inputs. Supported data types include Booleans, integers and floating or fixed-point numbers. Additionally, distance metrics such as Tanimoto similarity, cosine similarity, L1 norm, L2 norm, and L∞ norm are relevant to the drug matching problem and can be computed using two-party MPC. In general, MPC protocols rely on communication among involved parties which suffer from network delays. Furthermore, reduction of the communication overhead is performed by pre-processing the molecular inputs of the involved parties using partially homomorphic encryption, such as Paillier encryption and somewhat homomorphic encryption schemes. Herein, in an instance, Paillier encryption is a partial homomorphic encryption technique which allows addition of two ciphertexts and/or multiplication of a ciphertext by a plaintext number. Additionally, a somewhat homomorphic encryption technique supports select operation that is either addition or multiplication up to a certain complexity, but these operations can only be performed a set number of times. Moreover, Tanimoto similarity yields the most direct distance measure in the case of molecular fingerprints. Furthermore, it calculates the fraction of shared bits between molecular fingerprints in the range 0-1. Additionally, partially homomorphic encryption allows only select mathematical functions to be performed on encrypted values. This means that only one operation, either addition or multiplication can be performed an unlimited number of times on the ciphertext.

In the present disclosure, the procedure to compute the distance metric called Tanimoto similarity by combining using homomorphic encryption with the two-party MPC protocols is based on the assumption that first party has a private vector molecular fingerprint $v_1 = (x_1,...,x_n)$ and second party has a private vector molecular fingerprint $v_2 = (y_1,...,y_n)$ where $x_i$ and $y_i$ are non-negative integers (note that rational numbers can be scaled into integers). The Tanimoto similarity is defined as

$$D_{tan} = \frac{\langle v_1 , v_2 \rangle}{|v_1|^2 + |v_2|^2 - \langle v_1, v_2 \rangle}$$

The computation of $D_{tan}$, can be performed directly using MPC protocols with element-wise encryption on vector molecular fingerprint of the first party and vector molecular fingerprint of the second party. In order to reduce the communication overhead and speed up the computation in the two-party MPC protocol, several optimisations are applied to pre-process the inputs of the first party and the second party. Furthermore, to compute $D_{tan}$ it is sufficient to compute

$$\delta = \frac{|v_1|^2 + |v_2|^2}{\langle v_1, v_2 \rangle},$$

since $D_{tan} = \frac{1}{\delta - 1}$. $D_{tan}$ Additionally, the intermediate value $a = |v_1|^2$ can be precomputed locally by the first party, while $b = |v_2|^2$ can be precomputed locally by the second party. Subsequently, partially homomorphic encryption (PHE) schemes with encryption or decryption algorithms (*enc,dec*) such as Paillier encryption, can be used to compute the value of $d = \langle v_1, v_2 \rangle$. Firstly, the first party encrypts the vector molecular fingerprint $v_1$ element-wise using their own public key and Paillier encryption. The result is $c_1 = (enc(x_1),...,enc(x_n))$ Subsequently, the first party sends $c_1$ to the second party. Secondly, using the homomorphic properties of Paillier encryption, the second party can perform the operations as mentioned in the present disclosure on $c_1$. The second party applies their vector molecular fingerprint $v_2$ to $c_1$ to obtain

$$c_2 = (enc(x_1 y_1), \ldots, enc(x_n y_n))$$

Subsequently, the second party computes the summation of elements in $c_2$ and obtains $c_3 = enc(d)$ with

$$d = x_1 y_1 + x_2 y_2 + \cdots + x_n y_n$$

Thirdly, the second party chooses a uniform random $r$ to randomise $c_3$ and obtains a new ciphertext $c_4 = enc(d + r)$. Finally, the first party obtains $d + r$ by decrypting the ciphertext $c_4$ and using the secret key corresponding to the first party's encryption public key. Considering that $r$ is chosen randomly by the second party, $d + r$ reveals no molecular information about d to the first party. Following the pre-processing, the first party has molecular inputs $(a, d + r)$ and second party has molecular inputs $(b, r)$. The first party and the second party can jointly run the two-party MPC protocol to compute $\delta = \frac{a+b}{d}$. During comparison between $D_{tan}$ and a given threshold value, the actual value of $D_{tan}$ leaks partial molecular information on vector molecular fingerprints $v_1$ and $v_2$. In order to achieve a higher level of privacy, MPC protocols can be used to evaluate whether $D_{tan}$ is below or above a certain threshold value $p$, and only reveal the final comparison results, for instance, $D_{tan} \geq p$ or $D_{tan} < p$, without revealing any other information, for instance, without revealing the actual value $D_{tan}$. Furthermore, to speed up the computation, several optimisations can be performed. In an example, the value of comparison $D_{tan} \geq p$ is equal to that of

$$(1 + p) \langle v_1, v_2 \rangle \geq p \left( |v_1|^2 + |v_2|^2 \right)$$

Similar formulas can be derived for other comparison operations. Moreover, combining with the pre-processing mentioned prior on the first party's and second party's molecular inputs, the comparison result can be obtained by checking if

$$(1 + p)\, d \geq p\, (a + b)$$

using MPC protocols. Additionally, while performing optimisations on Boolean vectors, when $v_1$ and $v_2$ are bit-vectors, the representation of Tanimoto similarity can be simplified as shown below

$$D_{tan} = \frac{\langle v_1, v_2 \rangle}{|v_1|^2 + |v_2|^2 - \langle v_1, v_2 \rangle} = \frac{d}{a + b - d}$$

Wherein, d is the number of 1 bit that $v_1$ and $v_2$ have in common, a is Hamming weight of $v_1$ and *b* is Hamming weight of $v_2$. Furthermore, the value $a + b - d$ can be computed as $d + e$. Herein, d is the Hamming weight of $v_1 \wedge v_2$ and e is the Hamming weight of $v_1 \oplus v_2$. Subsequently, the two-party MPC protocols can compute and reveal the actual value of $D_{tan} = d/(d + e)$ or the comparison result $D_{tan} \geq p$ by checking if $(1 - p)\, d \geq p\, e$. Additionally, Paillier encryption is used to pre-compute the value of d and randomise it as $d + r_1$, as well as pre-compute the value of e and randomise it as $e + r_2$. Subsequently, the MPC protocols can be used to remove these randomisers $r_1$, $r_2$ to compute $D_{tan}$ or $D_{tan} \geq p$ . Moreover, when $v_1$ and $v_2$ are long vector molecular fingerprints, the pre-computation can reduce the computation and communication overhead for running the MPC protocols.

Optionally, the other types of distance metrics L1, L2, and L∞ norms for the distance between vectors can also be computed using the MPC protocols in a privacy-preserving manner. Additionally, these distance metrics start with an element-wise subtraction step. Herein, the L1 norm computes an element-wise absolute value followed by summation, and the L∞ norm involves element-wise comparisons. The L2 norm computes element-wise squaring followed by a sum operation, wherein the final square root does not need to be calculated using privacy-preserving computation Furthermore, an alternative way of computing the actual value of L2 norm is to use somewhat homomorphic encryption.

Various embodiments and variants disclosed in the present disclosure apply mutatis mutandis to the method.

The method comprises facilitation of inter-party communication relating to molecular fingerprints, the method comprising receiving a search query from a first party, the search query comprising molecular information pertaining to molecular structure of at least one molecule. Herein, the search query is a private molecular information that is checked against the data repository to find a possible match without leaking sensitive information to any of the parties involved. Additionally, the search query can also be a database entry by any one or multiple parties involved. Additionally, the present disclosure further comprises processing the molecular information to map the-molecular structure of a given molecule in the search query to a molecular fingerprint corresponding thereto using neural networks.

The method comprises matching the molecular fingerprint corresponding to the search query from the first party to an existing encrypted molecular fingerprint from a second party stored in a data repository. Notably, molecular graph matching technique is used which involves a computationally intensive interactive protocol that performs matching of more complex three-dimensional representation of the molecule which is most generally a molecular graph, between the query and a series of targets to establish full molecular identity between the query and the data repository. In an instance, the atoms in a molecule are considered as nodes and the bonds as edges. Notably, a molecular graph is a representation of a three-dimensional molecular information such as location of atoms, bond angles and/or chirality. Furthermore, any spatial relationship between the nodes must be encoded as node and/or edge attributes, as nodes in the molecular graph only have pairwise relationships.

In an embodiment, private molecular graph matching is performed using MPC protocols, wherein two parties holding respectively molecular graphs $G_1$ and $G_2$, wish to perform a computation on their joint molecular graph in a privacy-preserving manner, that is, without leaking any molecular information about their molecular inputs except that revealed by the computation's output. The instances include privacy-preserving computation of all pairs shortest distance, single source shortest distance, and maximum flow.

The method comprises performing operations on the existing encrypted molecular fingerprint for generating a ciphertext, wherein the ciphertext enables MPC between the first party and the second party by computing a function on the matched molecular fingerprints.

In an embodiment, encryption of the molecular fingerprints using a symmetric encryption algorithm with homomorphic properties is performed wherein, the molecules are passed through a processor function that maps the three-dimensional molecular structure to a series of molecular fingerprints, which are simple Boolean or integer vectors molecular fingerprints, $x_1$, $x_2$ upto $x_n$. Additionally, the vector molecular fingerprints are in the data repository of the first party. Furthermore, the first step of the protocol for computing the private function f(*.*) reveals no information about the private data repository and can be stored publicly on a shared and/or public database. Subsequently, privacy-preserving computation of molecular fingerprint matching is performed wherein, the second party takes a query molecule and constructs a molecular fingerprint, $y_j$, with an identical processor function to that used by the first party. The second party performs a cryptographic operation $op_2(pk,y_i$ to generate a ciphertext ($c_4$). Notably, the ciphertext can be used to perform a multi-party computation between the first party and the second party that computes a function on the vector molecular fingerprints, $f(x_i,y_j)$, without revealing the second party's query to the first party or any other molecular information about the data repository entry, $x_i$, to the second party. However, sometimes multi-party computation techniques can be replaced by customized homomorphic encryption techniques for better performance and greater simplicity.

The method comprises matching the molecular fingerprint corresponding to the search query from the first party to an existing encrypted molecular fingerprint from a second party stored in a data repository comprises using randomized simplified molecular-input line-entry system (SMILES) representation of molecules by either convolutional or recurrent neural network (RNN) layers. Notably, SMILES is a chemical notation that allows a party to represent a chemical structure in a way that can be used by the server arrangement. Furthermore, SMILES supports all elements in the periodic table, represents simple chain structures, branches, rings and also identifies charges on an atom. RNN are a special type of network for modelling sequence data: $x^{<t>} \rightarrow y^{<t>}$, where t is an index. These models introduce an internal memory, which is used for remembering previous molecular information in the sequence, and what is remembered is also a trained parameter. The update rules are the following:

$$a^{<0>} = 0, a^{<i+i>} = g(W_{aa}a^{<i>} + W_{ax}x^{<i+1>} + b_a)$$

$$y^{<i>} = g(W_{ya}a^{<i>} + b_y),$$

where *a* is the internal memory, *W,b* are learned weights and *g* is the activation function such as tanh activation function. However, simple RNN networks have a problem called vanishing gradient, which happens in cases where the molecular input sequences are long, due to backpropagation through time. Henceforth, long short-term memory (LSTM) units were introduced to solve this issue. The main idea is to make the molecular information flow $a^{<t>}$ through time linear, in which case the gradient won't decrease significantly. Similar to simple RNN cell, an internal state $c^{<t>}$ is introduced, but instead of having non-linear update rule, it is considered as a memory vector, and is updated linearly by introducing update gate ($\Gamma_u$) and forget gate ($\Gamma_f$). Thereby, the update rule for the state is

$$c^{<t>} = \Gamma_u \underline{c} + \Gamma_f c^{<t-1>}$$

where c is a new candidate value, calculated similarly as in RNN cell

$$\underline{c} = g(W_{cc}c^{<i-1>} + W_{cx}x^{<i>} + b_c).$$

Additionally, learnings from the new value and forgotten molecular information from the previous states are also learned parameters, with the following rules:

$$\Gamma_u = \sigma(W_{uc}c^{<i-1>} + W_{ux}x^{<i>} + b_u)$$

$$\Gamma_f = \sigma(W_{fc}c^{<i-1>} + W_{fx}x^{<i>} + b_f),$$

where the $\sigma$ is the Sigmoid function. The part of the internal state that goes to the output is decided by an output gate ($\Gamma_o$): $y^{<t>} = \Gamma_o g(c^{<t>})$, and similarly the output gate is learned

$$\Gamma_o = \sigma(W_{oc}c^{<i-1>} + W_{ox}x^{<i>} + b_o).$$

Therefore, these sequence models can be trained on randomized SMILES strings, which is a sequence of the characters.

Optionally, the requirement for inexact matching has motivated several different approaches to represent molecules comprising lists of atoms and positions, graphs of atoms, SMILES or various molecular fingerprints. In an instance, an efficient molecular fingerprinting scheme is Morgan molecular fingerprinting which represents the molecular structures by recursively partitioning the molecule into atomic neighborhoods. However, these representations result in the molecules being projected into a very high dimensional space that is not properly representative of the structural distance between molecules. Therefore, an important component of the present disclosure is the use of neural networks to generate compact representations of molecules that can be used for fuzzy private data repository queries. Subsequently, the approach is to build a representation of molecules which can be used for molecule matching. This is based on randomized SMILES representation of molecules, and uses either convolutional or RNN layers. Additionally, both adversarial or variational architecture can be used. Furthermore, the simplest implementation of convolutional or RNN layers for molecule matching is used for training in advance and agreed between multiple parties. Moreover, the parties have a mutual interest in developing a performant and interoperable technique, hence there are good reasons for construction of the data repository cooperatively and publicly. The techniques described in the present disclosure can be applied to the molecule in private and the molecular fingerprint's privacy is protected by homomorphic encryption or the MPC protocols as stated previously.

Optionally, the neural networks are also trained privately by one of the parties. Furthermore, the neural networks are obscured by homomorphic encryption and the molecular fingerprint data repository constructed by iterative application of this private neural network to a library of molecules. Additionally, the molecular fingerprints of the second party can be constructed similarly and the MPC protocols, as stated previously, can be used to construct private data repository queries. Additionally, the private neural network can be trained in a privacy-preserving way by a collection of nodes each holding their own data.

Optionally, the coordination of the different parties can be mediated by either a peer-to-peer network, public internet, cloud servers or blockchains. Furthermore, the multiple parties can be coordinated by rules encoded in smart contracts deployed on a blockchain. These smart contracts can include financial incentives and/or penalties to ensure, in an instance, that the holder of a data repository is available for processing queries using MPC. Moreover, the smart contracts could contain logic that ensures that excessive molecular information is not leaked to the outside world or to any of the other parties. Additionally, the data repositories could be split among multiple parties using secret sharing to ensure availability. The parties could have different roles such as, operating the blockchain network, hosting an encrypted data repository, performing computations or verifying the actions of other parties. The data repositories themselves could be stored in the cloud or on distributed file systems via the Inter Planetary File System (IPFS). Notably, the integrated cryptographic system can further be combined with laboratory information management systems to automate the drug discovery process.

Optionally, biopolymer sequences such as deoxyribonucleic acids, ribonucleic acids and/or protein sequences have molecular structures that are encoded entirely by their one-dimensional sequence, which makes them more straightforward to represent with molecular fingerprints than molecules. The general sequence molecular graph matching problem is simplified to a polynomial-time dynamic programming problem. A similar three-step protocol is proposed for these molecules. Firstly, direct molecular identity which can be established straightforwardly by computing cryptographic hashes of raw sequences. Secondly, Multiple Sequence Alignments (MSA) that can be used to align closely related sequences such as, antibodies or a specific type of surface antigen. Furthermore, as the general class of molecule is typically public knowledge, this MSA processing step can also be performed publicly, provided that a procedure is put in place to resolve distinct alignments with equivalently scored matches. Additionally, the MSA maps a collection of variable length sequences to a fixed-length reference vector molecular fingerprint including gaps. Moreover, these fixed-length vector molecular fingerprints can be compared using privacy-preserving techniques, similar to the ones described in the present disclosure, using summed Tanimoto distances on bit-strings representing an encoding of each character. Thirdly, global and local pairwise sequence alignments are used which are simplified versions of the general molecular graph matching problem but with constraints that allow them to be solved with dynamic programming such as Needleman-Wunsch algorithm and Smith-Waterman algorithms, which can run in quadratic time. Notably, MPC protocols provide support for describing algorithms in high-level languages such as C. These algorithms will then be converted into Boolean/arithmetic circuits in order to be computed on private molecular inputs from multiple parties using MPC protocol.

## DETAILED DESCRIPTION OF THE DRAWINGS

Referring to FIG. 1, there is shown an illustration of representation of complex three-dimensional structures of molecules using simplified representations, in accordance with an embodiment of the present disclosure. Notably, the presence of different functional groups within the molecule are used to create a binary vector that represents the drug molecule.

Referring to FIG. 2, there is shown an illustration of encryption algorithm for database, in accordance with an embodiment of the present disclosure. Notably, the molecules are passed through a processor that maps three-dimensional molecular structure to a series of molecular fingerprints, which are Boolean or integer vectors, $x_1, ..., x_n$. These molecular fingerprints are encrypted with a strong symmetric encryption algorithm that can be stored in a public database without leaking any of first party's, such as Alice's, private information.

Referring to FIG. 3, there is shown an illustration of multi-party computation of molecular fingerprint matching, in accordance with an embodiment of the present disclosure. Notably, a second party, such as Bob, takes a query molecule and constructs a molecular fingerprint, $y_j$, with an identical processor to that used by the first party. The second party performs operations on an encrypted entry in the first party's database, $enc(x_i)$ to generate a ciphertext ($c_4$). This ciphertext can be used to perform a multi-party computation between the first party and the second party that computes a function on the vectors, $f(x_i,y_j)$, without revealing the second party's query to the first party or any other information about the database entry, $x_i$, to the second party.

Referring to FIG. 4, there is shown an illustration of representation of the calculation rules of a recurrent neural networks (RNN), in accordance with an embodiment of the present disclosure. Notably, RNN are a special type of networks for modelling sequence data: $x^{<t>} \rightarrow y^{<t>}$, where t is an index. These RNN models introduce an internal memory, which is used for remembering previous molecular information in the sequence, and what is remembered is also a trained parameter. Subsequently, the update rules are

$$a^{<0>} = 0, a^{<i+i>} = g(W_{aa}a^{<i>} + W_{ax}x^{<i+1>} + b_a)$$

$$y^{<i>} = g(W_{ya}a^{<i>} + b_y),$$

where a is the internal memory, *W,b* are learned weights and g is the activation function such as tanh activation function.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

**1.** A server arrangement arranged to process molecular information, the server arrangement configured to

- receive an input of the molecular information, wherein the molecular information comprises information pertaining to a molecular structure of at least one molecule;
- process the molecular information to map the molecular structure of each of the at least one molecule in the input to a molecular fingerprint corresponding thereto using neural networks, wherein the molecular fingerprint is a representation of the at least one molecule in a multi-dimensional space that enables comparison of the at least one molecule with other molecules;
- encrypt the molecular fingerprints using a symmetric encryption algorithm with homomorphic properties; and
- store the encrypted molecular fingerprints in a data repository.

**2.** A system of claim 1, wherein the server arrangement is configured to receive the input from a user or a database storing the molecular information.

**3.** A system of claim 2, wherein the neural networks for mapping the molecular structure comprise at least one of

- variational autoencoders wherein the molecular information is represented as distributions over the latent space instead of single points;
- adversarial autoencoders wherein an autoencoder network encodes the input into a low dimensional embedding space and a discriminator network predicts whether the embedding is from an encoder or from a normal distribution.

**4.** A system of any of the preceding claims, wherein the server arrangement is configured to receive input of biopolymer sequences such as deoxyribonucleic acid sequence, ribonucleic acid sequence and/or protein sequence.

**5.** A method of facilitating inter-party communication relating to molecular fingerprints, the method comprising

- receiving a search query from a first party, the search query comprising molecular information pertaining to molecular structure of at least one molecule;
- processing the molecular information to map the molecular structure of a given molecule in the search query to a molecular fingerprint corresponding thereto using neural networks;
- matching the molecular fingerprint corresponding to the search query from the first party to an existing encrypted molecular fingerprint from a second party stored in a data repository;
- performing operations on the existing encrypted molecular fingerprint for generating a ciphertext, wherein the ciphertext enables multi-party computation between the first party and the second party by computing a function on the matched molecular fingerprints.

**6.** A method of claim 5, wherein matching the molecular fingerprint corresponding to the search query from the first party to an existing encrypted molecular fingerprint from a second party stored in a data repository comprises using randomized simplified molecular-input line-entry system representation of molecules by either convolutional or RNN layers.

**7.** A method of any of claims 5 or 6, wherein the encrypted molecular fingerprint in the data repository is trained by the neural networks in a privacy preserving manner by a collection of nodes each holding their own dataset.

**8.** A method of processing molecular information, the method comprising

- receiving an input of the molecular information, wherein the molecular information comprises information pertaining to molecular structure of at least one molecule;
- processing the molecular information to map the molecular structure of each of the at least one molecule in the input to a molecular fingerprint corresponding thereto using neural networks, wherein the molecular fingerprint is a representation of the at least one molecule in a multi-dimensional space that enables comparison of the at least one molecule with other molecules;
- encrypting the molecular fingerprints using a symmetric encryption algorithm with homomorphic properties; and
- storing the encrypted molecular fingerprints in a data repository.

**9.** A software product recorded on machine-readable non-transient data storage media, wherein the software product is executable upon computing hardware to implement a method of any of the claims 5 to 8.

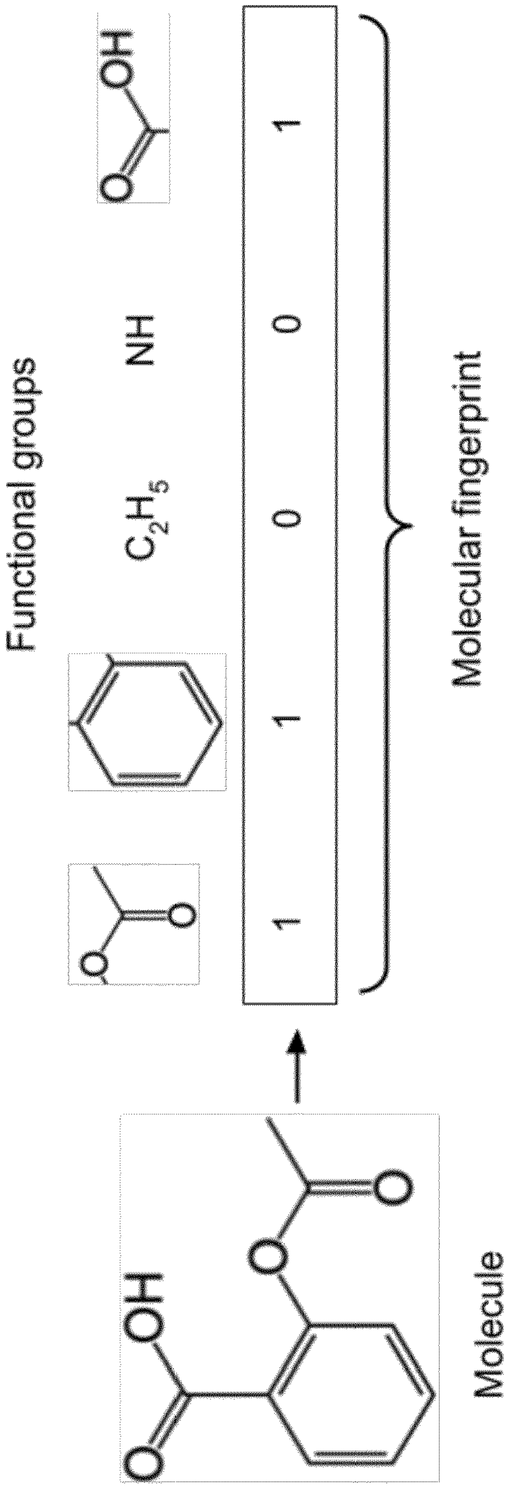
Functional groups
$C_2H_5$
NH
1
1
0
0
1
Molecular fingerprint
Molecule

FIG. 1

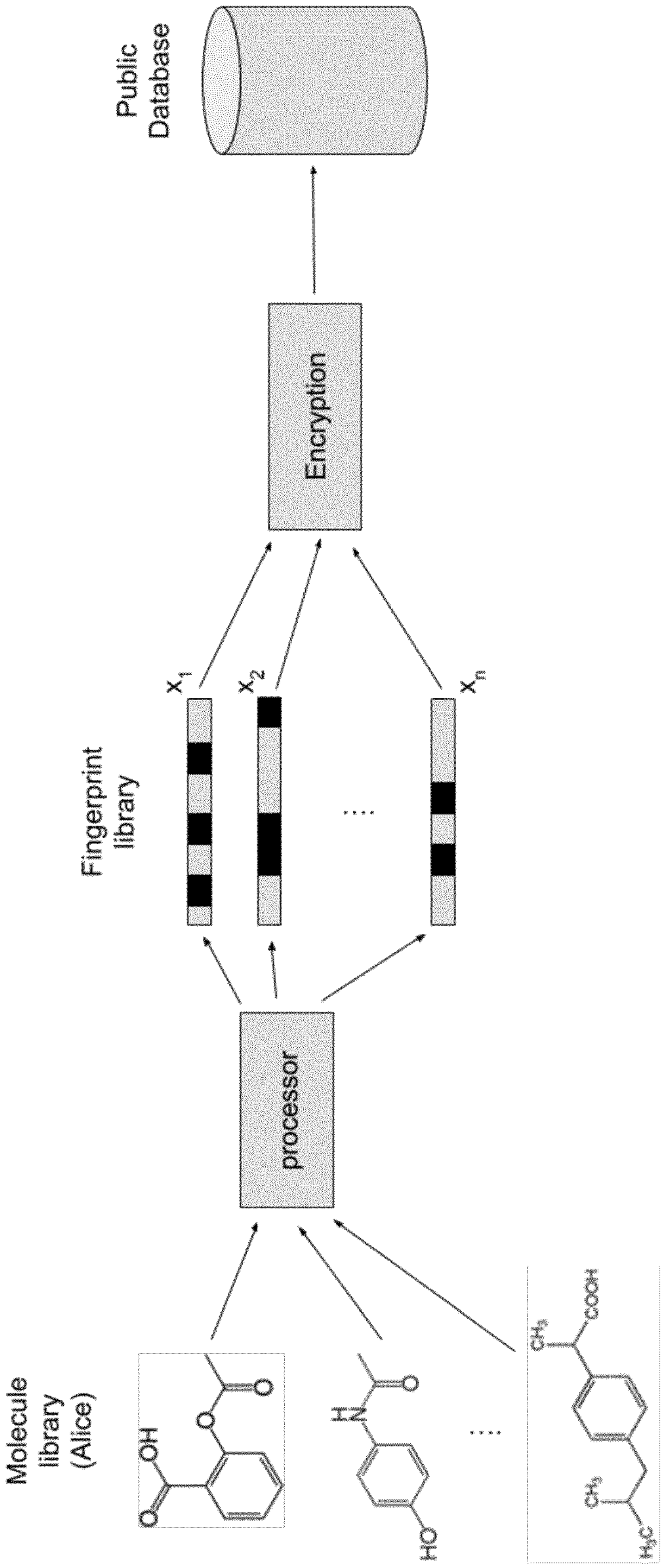
Molecule library (Alice)
....
processor
Fingerprint library
x1
x2
....
xn
Encryption
Public Database

FIG. 2

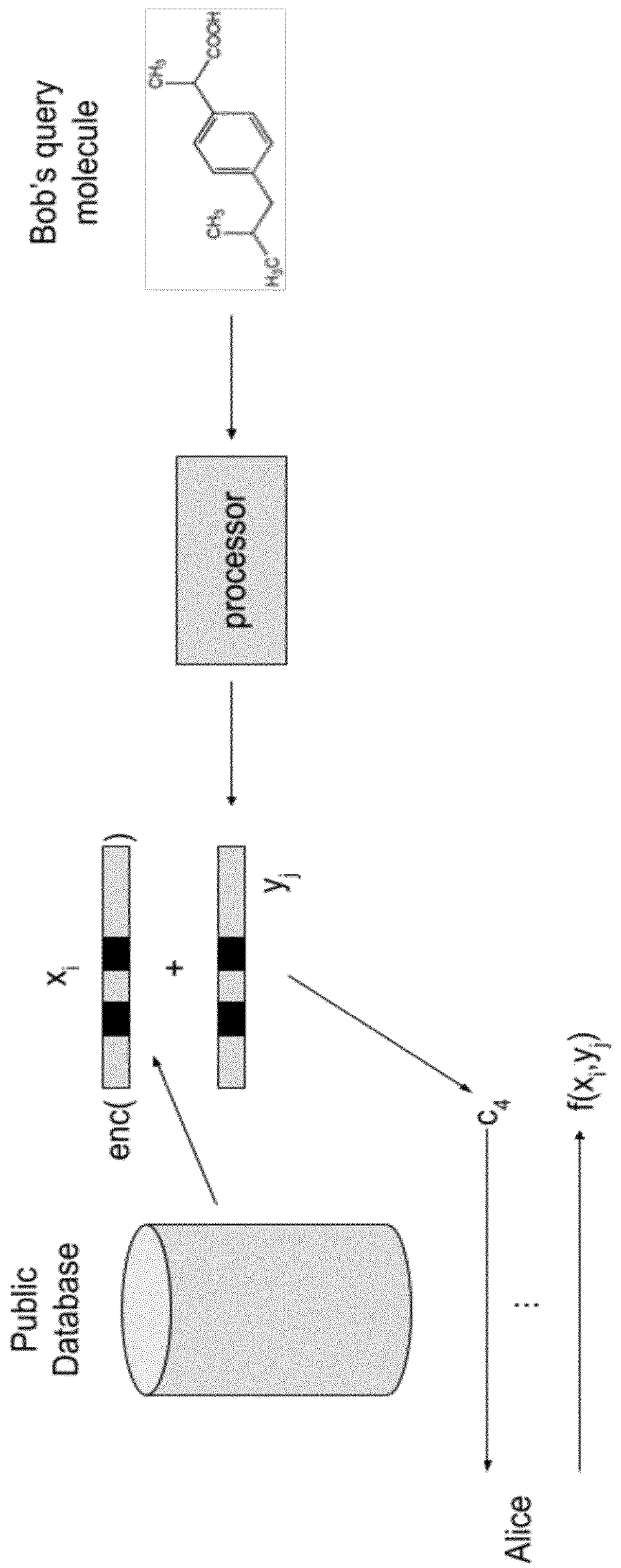
Bob's query molecule
processor
Public Database
enc(
)
+
$x_i$
$y_j$
$c_4$
$f(x_i,y_j)$
...
Alice

FIG. 3

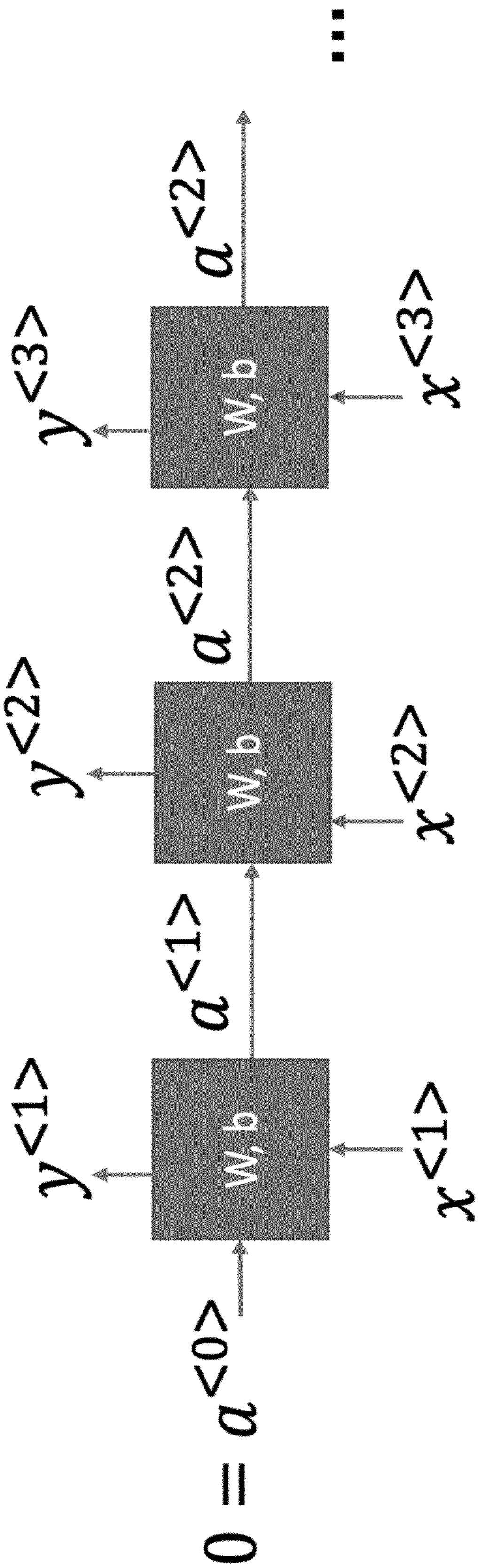


FIG. 4

Europäisches Patentamt
European Patent Office
Office européen des brevets

# EUROPEAN SEARCH REPORT

Application Number
EP 21 16 2297

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2014/355756 A1 (IWAMURA KANA [JP] ET AL) 4 December 2014 (2014-12-04)<br>* whole document, in particular figures 1-4 and 6; paragraphs [0001], [0010]-[0011], [0040]-[0058], [0077], [0102] and [0134]; claims *<br>----- | 1-9 | INV.<br>G16B20/30<br>G16B40/30<br>G16C20/20 |
| Y | MRIGANKA NATH ET AL: "Toxicity Detection in Drug Candidates using Simplified Molecular-Input Line-Entry System", ARXIV.ORG, CORNELL UNIVERSITY LIBRARY, 201 OLIN LIBRARY CORNELL UNIVERSITY ITHACA, NY 14853,<br>21 January 2021 (2021-01-21), XP081867484, DOI: 10.5120/IJCA2020920695<br>* Whole document, in particular paragraphs 1-4 on pages 1-2 *<br>----- | 1-9 | |
| Y | WOOD ALEXANDER ANWOOD@MED UMICH EDU ET AL: "Homomorphic Encryption for Machine Learning in Medicine and Bioinformatics", ACM COMPUTING SURVEYS, ACM, NEW YORK, NY, US, US,<br>vol. 53, no. 4,<br>25 August 2020 (2020-08-25), pages 1-35, XP058487640,<br>ISSN: 0360-0300, DOI: 10.1145/3394658 | 4,7 | TECHNICAL FIELDS SEARCHED (IPC) |
| A | * whole document, in particular paragraph "Fully homomorphic encryption for bioinformatics" on pages 21-26; introduction and figures *<br>----- | 1-3,5,6, 8,9 | G16B<br>G16C |
| Y | US 2020/327250 A1 (WANG SHUANG [US] ET AL) 15 October 2020 (2020-10-15) | 4 | |
| A | * whole document, in particular claims ; figures 9, 10 and 30 ; paragraphs 372-373 , 379 , 417, 434 - 436 *<br>----- | 1-3,5-9 | |

The present search report has been drawn up for all claims

2

| Place of search | Date of completion of the search | Examiner |
|---|---|---|
| Munich | 20 August 2021 | Vanmontfort, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 16 2297

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2021

| Patent document cited in search report | Publication date | Patent family member(s) | Publication date |
|---|---|---|---|
| US 2014355756 A1 | 04-12-2014 | JP 5975490 B2 | 23-08-2016 |
| | | JP WO2013038698 A1 | 23-03-2015 |
| | | US 2014355756 A1 | 04-12-2014 |
| | | WO 2013038698 A1 | 21-03-2013 |
| US 2020327250 A1 | 15-10-2020 | CN 113169957 A | 23-07-2021 |
| | | US 2020327250 A1 | 15-10-2020 |
| | | WO 2020206695 A1 | 15-10-2020 |

EPO FORM P0459

For more details about this annex : see Official Journal of the European Patent Office, No. 12/82